# EUROPEAN PATENT APPLICATION

(11) **EP 4 596 716 A1**
(43) Date of publication of application: **06.08.2025**
(21) Application number: 22960996.1
(22) Date of filing: 30.09.2022
(51) Int. Cl.: C12Q 1/6844, C12M 1/00

(54) **REACTION VESSEL FOR NUCLEIC ACID AMPLIFICATION, CARTRIDGE, AND NUCLEIC ACID AMPLIFICATION METHOD**

(71) Applicant: Kabushiki Kaisha Mirai Genomics, Yokohama-shi, Kanagawa 230-0051 (JP)
(72) Inventor: OVECHKIN Nikita, Yokohama-shi, Kanagawa 230-0051 (JP); PUZANKOV Dmitrii, Yokohama-shi, Kanagawa 230-0051 (JP)
(74) Representative: f & e patent
(86) International application number: PCT/JP2022/036722
(87) International publication number: WO 2024/069937

(57) **Abstract**

The present invention relates to a reaction vessel for nucleic acid amplification with a vertically long shape, including a reaction solution storage space, which is a lower portion thereof in a longitudinal direction, and a reaction solution supply space, which is an upper portion thereof, and comprising a reaction solution supply port and an exhaust port in the reaction solution supply space, wherein the reaction solution supply port is positioned below the exhaust port in the longitudinal direction. Also regarding a reaction vessel for nucleic acid amplification with a vertically long shape, including a reaction solution storage space, which is a lower portion thereof in a longitudinal direction, and a reaction solution supply space, which is an upper portion thereof, and comprising a protrusion toward the inside of the reaction vessel, in the vicinity of at least a part of the boundary between the reaction solution supply space and the reaction solution storage space. The present invention provides a reaction vessel for nucleic acid amplification, a cartridge, and a nucleic acid amplification method capable of suppressing an occurrence of false positive.

## Description

### TECHNICAL FIELD

The present invention relates to a reaction vessel for nucleic acid amplification, a cartridge, and a nucleic acid amplification method.

### BACKGROUND ART

With the spread of COVID-19, there is a growing demand for technologies to recover, amplify and detect nucleic acid in viruses to conveniently and reliably determine the presence or absence of viral infection. In addition, even after COVID-19 problem has been resolved, human beings continue to face diseases caused by various viruses and bacteria, and therefore demands for similar technologies still remain.

The technology for amplifying and detecting nucleic acid has been almost established as a methodology thanks to the advent of PCR method with thermocyclers and the subsequent development of various methods other than thermocycle PCR method, and has become convenient enough to be used not only in laboratories but also in examination rooms in hospitals. Even so, in the case of using existing devices for amplification and detection, the amplification and detection of nucleic acid were often performed after performing manual operations to recover nucleic acid from biological samples such as viruses. However, with the spread of COVID-19, it became clear that a serious situation cannot be dealt with by using existing methods and devices for specimens brought to public health centers or examination companies. At present, it is not possible to reliably determine the presence or absence of viral infection by processing a large amount of specimens quickly and simply, regardless of location.

Patent Literature 1 discloses a portable small-sized device for amplification and detection that can more easily amplify and detect nucleic acid.

Patent Literature 1: International Publication WO 2018/123837

The entire description of Patent Literature 1 is incorporated herein by reference in particular.

### SUMMARY OF INVENTION

### TECHNICAL PROBLEM

The device described in Patent Literature 1 is a device that separately recovers nucleic acid from a specimen and then amplifies and detects the recovered nucleic acid. However, in the method, it is necessary to separately recover the nucleic acid from the specimen, and it is not possible to detect viruses from the specimen at once.

In contrast, the present inventors planned to use a specimen as it is without pretreating, and to perform recovering nucleic acid from the specimen, amplifying the recovered nucleic acid, and detecting the amplified nucleic acid, in a single device. This device is intended to perform the above-mentioned step in chambers interconnected by micro flow channels in a sequential and automatic manner, but the present inventors have found that the false positive may occur due to the reaction vessel for nucleic acid amplification, in which the recovered nucleic acid is amplified.

An object of the present invention is to provide a reaction vessel for nucleic acid amplification capable of suppressing the occurrence of the false positive, a cartridge for nucleic acid amplification comprising the same, and a nucleic acid amplification method with the cartridge, and the present invention aims to provide a reaction vessel for nucleic acid amplification capable of suppressing the occurrence of the false positive, a cartridge for nucleic acid amplification comprising the same, and a nucleic acid amplification method with the cartridge.

In the case of detecting viruses in a specimen with a cartridge having a plurality of chambers interconnected by micro flow channels, the smaller the amount of nucleic acid amplification reaction solution is, the lower the necessary amount of enzymes and primers can be reduced, and the higher the concentration of the amplified nucleic acid can be increased, so the possibility of improving sensitivity increases. On the other hand, the smaller the amount of the reaction solution, the more difficult it is to position the reaction solution in a predetermined position within the reaction vessel, usually on the bottom, due to the interaction between the interfacial tension of the solution and the inner surface. In particular, in a method in which the reaction solution is supplied from the reaction solution supply port of the reaction vessel to the reaction solution supply space by a suction from an exhaust port to the outside, bubbles are generated at the time of supplying the reaction solution, and if the bubbles remain, the reaction solution will not be positioned in the predetermined position, and this situation can cause the false positive in amplification and detection of nucleic acid. This trend is particularly pronounced when the volume of the reaction solution is, for example, 10 to 500 µL, 10 to 100 µL.

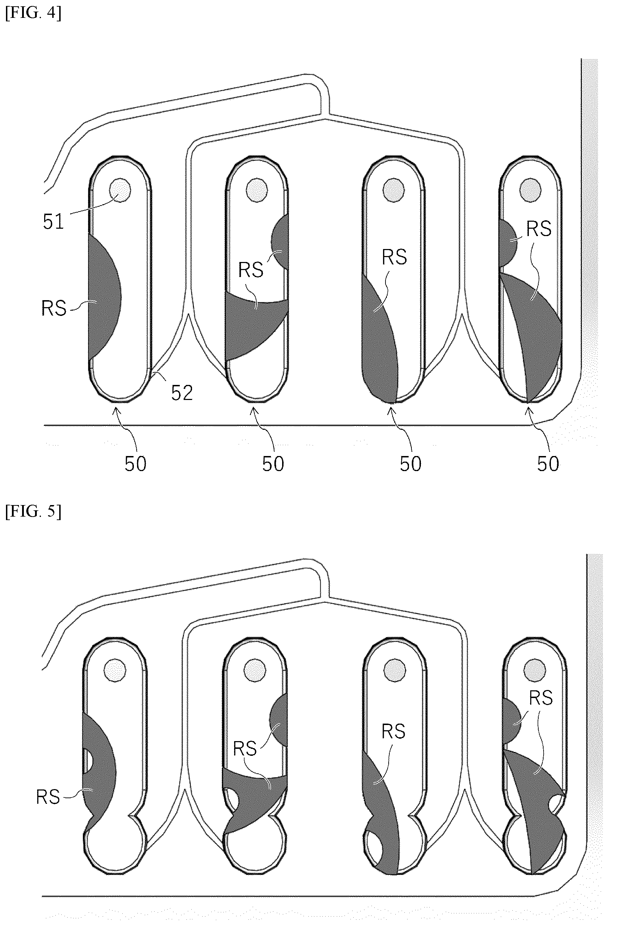

FIG. 4 is a diagram of a reaction vessel 50 with a vertically long shape, having an exhaust port 51 in the upper portion and having a reaction solution supply port 52 near the bottom, showing a situation in which reaction solution RS is retained on a wall surface in the reaction vessel and reaction solution RS cannot be stored in a reaction solution storage space, which is the lower portion in the longitudinal direction. The reaction solution is transferred into the reaction vessel 50 by generating negative pressure inside the vessel by a suction from the exhaust port 51, and then suctioning the reaction solution from a separate chamber into the supply port near the bottom. However, since the reaction solution scatters in the reaction vessel 50, as shown in FIG. 4, the reaction solution may adhere to the wall surface of the reaction vessel to become bubble-like.

In order to eliminate the bubble-like reaction solution adhering to the wall surface in the reaction vessel and to store the reaction solution in the predetermined position in the reaction vessel, in one aspect of the present invention, the reaction vessel has a reaction solution supply port and an exhaust port in the reaction solution supply space, and the reaction solution supply port is positioned below the exhaust port in the longitudinal direction of the reaction vessel. The reaction solution supply port is positioned below the exhaust port, so the reaction solution supplied from the reaction solution supply port easily flows downward, and the bubble easily escapes upward.

Furthermore, in order to eliminate the bubble-like reaction solution adhering to the wall surface in the reaction vessel and to store the reaction solution in the predetermined position in the reaction vessel, the present invention provides, in another aspect, a protrusion toward the inside on at least a part of the boundary between the lower portion and the upper portion of the reaction vessel. The bubble tends to disappear at the tip of the protrusion, and as a result, the solution tends to collect at the bottom.

Furthermore, in the present invention, it was found that by positioning the reaction solution supply port below the exhaust port and above the protrusion, rather than near the bottom, the bubble generation can be suppressed more, and the reaction solution can be stored more easily in the space below the protrusion, and as a result, the occurrence of the false positive due to the reaction solution not being in the predetermined position can be suppressed more.

### SOLUTION TO PROBLEM

The present invention is as follows.
[1] A reaction vessel for nucleic acid amplification with a vertically long shape, including a reaction solution storage space, which is a lower portion thereof in a longitudinal direction, and a reaction solution supply space, which is an upper portion thereof, and comprising a reaction solution supply port and an exhaust port in the reaction solution supply space,
   wherein the reaction solution supply port is positioned below the exhaust port in the longitudinal direction.
[2] The reaction vessel according to [1], used for a method in which the reaction solution is supplied from the reaction solution supply port to the reaction solution supply space by a suction from the exhaust port to the outside.
[3] A reaction vessel for nucleic acid amplification with a vertically long shape, including a reaction solution storage space, which is a lower portion thereof in a longitudinal direction, and a reaction solution supply space, which is an upper portion thereof, and comprising a protrusion toward the inside of the reaction vessel, in the vicinity of at least a part of the boundary between the reaction solution supply space and the reaction solution storage space.
[4] The reaction vessel according to [1] or [2], wherein the reaction vessel comprises a protrusion toward the inside of the reaction vessel, in the vicinity of at least a part of the boundary between the reaction solution supply space and the reaction solution storage space.
[5] The reaction vessel according to [3] or [4], wherein the protrusion is present along the entire boundary.
[6] The reaction vessel according to any one of [3] to [5], wherein a ratio of a horizontal cross-sectional area at the position where the protrusion is provided is in the range of 30 to 95 when a horizontal cross-sectional area of the reaction vessel is set to 100.
[7] The reaction vessel according to any one of [3] to [6], wherein an opening area in a horizontal cross-section at the position where the protrusion is provided is 1 to 10 mm².
[8] The reaction vessel according to any one of [4] to [7], in the case that the reaction vessel has an exhaust port in the reaction solution supply space,
   the reaction solution supply port is positioned below the exhaust port and above the protrusion.
[9] The reaction vessel according to any one of [1] to [8], wherein the reaction solution storage space has a volume in the range of 10 to 500 µL.
[10] The reaction vessel according to any one of [4] to [9], in the case that the reaction vessel has an exhaust port in the reaction solution supply space,
   L1:L2:L3 ratio can be, for example, in the range of 1:0.1 to 1:0.1 to 1, wherein L1 is a distance between the exhaust port and the reaction solution supply port, L2 is a distance between the protrusion and the reaction solution supply port, and L3 is a distance between the protrusion and the bottom of the reaction solution storage space, and when L1+L2+L3 is set to 1, L2+L3 is, for example, in the range of 0.3 to 0.7.
[11] The reaction vessel according to any one of [1] to [10], wherein at least a part of the side wall of the reaction solution storage space has light transparency.
[12] The reaction vessel according to any one of [1] to [11], wherein the reaction solution storage space has a horizontal cross-sectional area smaller than a horizontal cross-sectional area of the reaction solution supply space.
[13] The reaction vessel according to any one of [1] to [12], wherein at least a part or all of the inner surface of the reaction vessel has a surface roughness Ra of 25 nm or less.
[14] The reaction vessel according to any one of [1] to [12], wherein at least a part or all of the inner surface of the reaction vessel is coated with wax.
[15] A cartridge (10) for nucleic acid amplification comprising chambers in communication via a micro flow channel, and at least one reaction vessel (20) for nucleic acid amplification in communication with the chambers via a micro flow channel (30 and 31),
   wherein the reaction vessel (20) is the reaction vessel according to any one of [1] to [14].
[16] A nucleic acid amplification method with the cartridge according to [15], comprising:
   generating negative pressure in the reaction vessel by a suction from the exhaust port and thereby supplying the reaction solution containing nucleic acid to be amplified from the reaction solution supply port to the reaction solution supply space,
   subjecting the reaction solution that has spontaneously moved from the reaction solution supply space to the reaction solution storage space, to a nucleic acid amplification reaction.

### ADVANTAGOUS EFFECT OF INVENTION

According to the present invention, it is possible to suppress the occurrence of the false positive in the detection after the nucleic acid amplification.

### BRIEF DESCRIPTION OF DRAWINGS

[FIG. 1] FIG. 1 is a schematic explanatory diagram of a portion of a cartridge of the present invention having an example reaction vessel.
[FIG. 2] FIG. 2 is a schematic explanatory diagram of a reaction vessel according to an example of the present invention.
[FIG. 3-1] FIG. 3-1 is a schematic explanatory diagram of a reaction vessel according to an example of the present invention.
[FIG. 3-2] FIG. 3-2 is a schematic explanatory diagram of a reaction vessel according to an example of the present invention.
[FIG. 4] FIG. 4 is a schematic explanatory diagram of a reaction vessel that is not the present invention.
[FIG. 5] FIG. 5 is a schematic explanatory diagram of a reaction vessel according to an example of the present invention.

### EMBODIMENTS OF INVENTION

### <Reaction Vessel for Nucleic Acid Amplification>

The reaction vessel for nucleic acid amplification according to the first aspect of the present invention relates to a reaction vessel for nucleic acid amplification with a vertically long shape, including a reaction solution storage space, which is a lower portion thereof in a longitudinal direction, and a reaction solution supply space, which is an upper portion thereof, and comprising a reaction solution supply port and an exhaust port in the reaction solution supply space, wherein the reaction solution supply port is positioned below the exhaust port in the longitudinal direction.

The reaction vessel for nucleic acid amplification according to the second aspect of the present invention relates to a reaction vessel for nucleic acid amplification with a vertically long shape, including a reaction solution storage space, which is a lower portion thereof in a longitudinal direction, and a reaction solution supply space, which is an upper portion thereof, and comprising a protrusion toward the inside of the reaction vessel, in the vicinity of at least a part of the boundary between the reaction solution supply space and the reaction solution storage space.

A preferred reaction vessel for nucleic acid amplification of the present invention relates to a reaction vessel with a vertically long shape comprising a common part of the first and second aspects, that is, including a reaction solution storage space, which is a lower portion thereof in a longitudinal direction, and a reaction solution supply space, which is an upper portion thereof, wherein the reaction vessel comprises a reaction solution supply port and an exhaust port in the reaction solution supply space, and the reaction solution supply port is positioned below the exhaust port in the longitudinal direction, and wherein the reaction vessel comprises a protrusion toward the inside of the reaction vessel, in the vicinity of at least a part of the boundary between the reaction solution supply space and the reaction solution storage space.

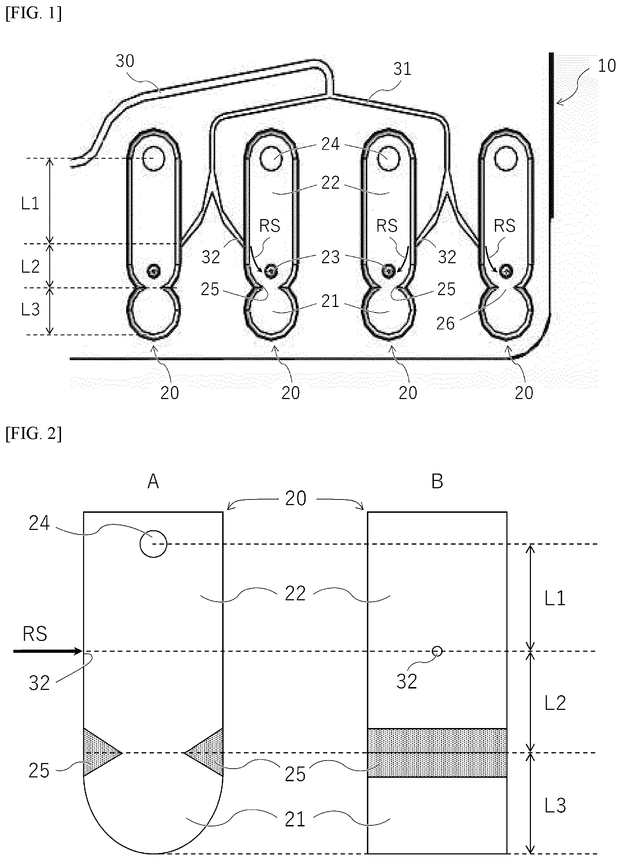

FIG. 1 and FIG. 2 are schematic explanatory diagrams of an example of a reaction vessel for nucleic acid amplification of the present invention. The reaction vessel for nucleic acid amplification of the present invention will be described with reference to FIGS. 1 and 2.

The reaction vessel 20 for nucleic acid amplification of the present invention is a reaction vessel with a vertically long shape, including a reaction solution storage space 21, which is a lower portion thereof in a longitudinal direction, and a reaction solution supply space 22, which is an upper portion thereof. The reaction solution supply space 22 has a reaction solution supply port 32 and an exhaust port 24. The reaction vessel comprises a protrusion 25 toward the inside of the reaction vessel, in the vicinity of at least a part of the boundary between the reaction solution supply space 22 and the reaction solution storage space 21. Section A of FIG. 2 shows a cross-section in the same plane as FIG. 1, and section B shows a cross-section of the reaction vessel 20 when rotated by 90° with respect to the vertical axis (longitudinal axis). From these figures, it can be seen that the space up to the protrusion 25 of the reaction solution supply space 22 is a rectangular parallelepiped. However, the reaction solution supply space 22 may be cylindrical, and the protrusion 25 may have a circular shape with a circular opening at the center thereof. The reaction solution supply space 22 may have a pillar 23 extending from the side wall in the reaction vessel 20 in the vicinity of the reaction solution supply port 32 or the protrusion 25.

The reaction solution storage space is a space for storing the reaction solution and amplifying the nucleic acid contained in the reaction solution, and the volume thereof is not particularly limited, but is, for example, in the range of 10 to 500 µL, preferably in the range of 10 to 100 µL, from the viewpoint that the nucleic acid in a trace amount of sample can be amplified and detected with high sensitivity. However, it is not intended to be limited to this numerical range.

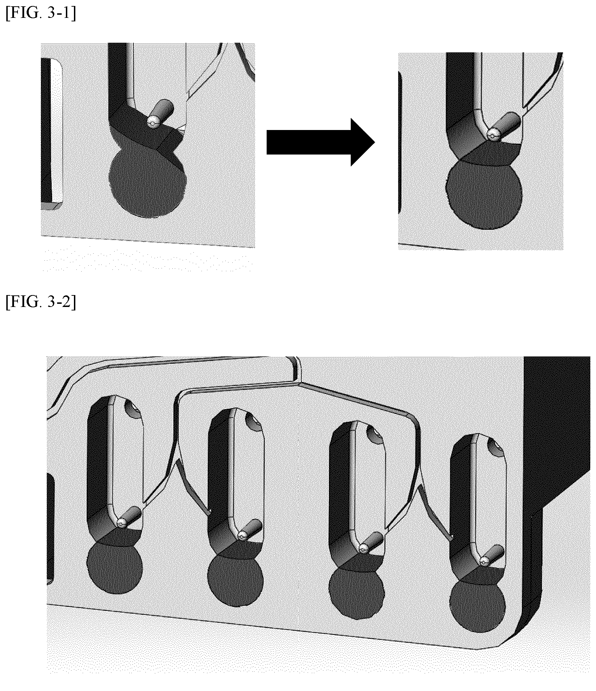

It is preferable that the protruding 25 in the vicinity of the boundary between the reaction solution supply space 22 and the reaction solution storage space 21 is present on a part or all of the boundary, or on all of the boundary. Due to the presence of the protrusion 25, the bubble generation at the time of supplying the reaction solution can be more easily eliminated, and the reaction solution can be easily stored in the reaction solution storage space 21. Furthermore, an opening 26 formed by the protrusion 25 serves as an entrance through which the reaction solution supplied to the reaction solution supply space 22 moves to the reaction solution storage space 21, and has a function of determining an area where a liquid level of the reaction solution after moving to the reaction solution storage space 21 comes into contact with the air in the reaction solution supply space 22. When the amount of the reaction solution is set to an amount in which the liquid level of the reaction solution is located at substantially the same height as the opening 26, the liquid level of the reaction solution has substantially the same area as that of the opening 26, and thereby becomes smaller than in the case where the protrusion 25 is not provided, resulting in that the reaction solution is easily stopped in the reaction solution storage space 21. As shown in FIG. 3-1, when the protrusion 25 is provided, the reaction solution tends to stop in the reaction solution storage space 21 so as to minimize the surface area by the interfacial tension.

When a horizontal cross-sectional area of the reaction vessel (for example, the cross-sectional area at the position where the reaction solution supply port 32 is provided) is set to 100, a ratio of a horizontal cross-sectional area at the position where the protrusion is provided, that is, the area of the opening 26, is preferably in the range of 30 to 95, and more preferably in the range of 60 to 80. The area of the opening 26 in the horizontal cross-section at the position where the protrusion 25 is provided depends on the horizontal cross-sectional area of the reaction vessel, but can be, for example, in the range of 1 to 10 mm². The horizontal cross-sectional area of the reaction vessel can be, for example, in the range of 2 to 20 mm². However, it is not intended to be limited to these numerical ranges.

It is preferable that the reaction solution supply port 32 is positioned below the exhaust port 24 and above the protrusion 25 from the viewpoint that the reaction solution is easily stored in the reaction solution storage space 21 as a single mass. When the reaction solution supply port 32 is positioned in the reaction solution storage space 21, the exhaust port 24 is positioned in the vicinity of the uppermost portion, and therefore, as shown in FIG. 5, the reaction solution supplied from the reaction solution supply port 32 stops in a space other than the reaction solution storage space 21 and tends to be difficult to be stored in the reaction solution storage space 21 as a single mass. As shown in FIG. 2, L1:L2:L3 ratio can be, for example, in the range of 1:0.1 to 1:0.1 to 1, or in the range of 1:0.3 to 0.8:0.3 to 0.8, wherein L1 is a distance between the exhaust port 24 and the reaction solution supply port 32, L2 is a distance between the protrusion 25 and the reaction solution supply port 32, and L3 is a distance between the protrusion 25 and the bottom of the reaction solution storage space 21. When L1+L2+L3 is set to 1, L2+L3 can be, for example, in the range of 0.3 to 0.7. However, it is not intended to be limited to these numerical ranges.

From the viewpoint of detecting the amplified nucleic acid as it is after the nucleic acid amplification or during the amplification, it is preferable that at least a part of the side wall of the reaction solution storage space has light transparency. In addition, the reaction solution storage space can have a horizontal cross-sectional area (e.g., the maximum cross-sectional area) smaller than a horizontal cross-sectional area of the reaction solution supply space. In particular, in the case that at least a part of the side wall has light transparency, it is preferable to make the horizontal cross-sectional area of the reaction solution storage space smaller than the horizontal cross-sectional area of the reaction solution supply space by shortening the distance between inner surfaces of the side wall having light transparency and a wall facing it. The amount of the reaction solution to be stored in the reaction solution storage space can be reduced, and detection light (excitation light) to the reaction solution containing the amplified nucleic acid can be efficiently utilized.

It is more preferable that at least a part or all of the inner surface of the reaction solution storage space 21 has a surface roughness Ra of 25 nm or less from the viewpoint of further suppressing the occurrence of the false positive. It is more preferable that at least a part or all of the inner surface of the reaction solution supply space 22 has a surface roughness Ra of 25 nm or less from the viewpoint of further suppressing the occurrence of the false positive. However, it is not intended to be limited to this numerical range.

It is preferable that at least a part or all of the inner surface of the reaction solution storage space 21 is coated with wax from the viewpoint of controlling the surface roughness and further suppressing the occurrence of the false positive. It is also preferable that at least a part or all of the inner surface of the reaction solution supply space 22 is coated with wax from the viewpoint of further suppressing the occurrence of the false positive.

### <Cartridge for Nucleic Acid Amplification>

The present invention relates to a cartridge 10 for nucleic acid amplification comprising chambers in communication via a micro flow channel, and at least one reaction vessel 20 for nucleic acid amplification in communication with the chambers via a micro flow channel 30 and 31, wherein the reaction vessel 20 is the reaction vessel of the present invention.

There is no particular limitation on the method of communicating the chambers via a micro flow channel and the structure of each micro flow channel. The number of reaction vessel 20 included in the cartridge of the present invention may be at least one, or at least two, such as 3, 4, 5, 6, 7, 8, 9, or 10, but is not limited to these numbers. FIG. 1 shows an example of a cartridge having four reaction vessels 20. The micro flow channel 30 from the upstream chamber is branched into two in the midstream to become the micro flow channels 31, each micro flow channel 31 is further branched into two to form four flow channels in total, these flow channels are in communication with the reaction solution supply ports 32 of the reaction vessels, respectively. The supply of the reaction solution to the reaction vessels 20 via the micro flow channel 30 and 31 is performed by applying negative pressure to the reaction solution supply space 22 from the exhaust port 24 located in the reaction solution supply space 22. The negative pressure can be applied to the exhaust port 24 by connecting, for example, a decompression pump. The decompression pump can be provided separately from the cartridge.

Even in the case that the cartridge of the present invention has at least two reaction vessels 20, if the reaction vessels 20 are the reaction vessels of the present invention, the reaction solution can be stored in the predetermined position within any of the reaction vessels. For example, even in the cartridge having four reaction vessels shown in FIG. 3-2, the reaction solution can be stored in the predetermined position within any of the reaction vessels.

### <Nucleic Acid Amplification Method>

The present invention includes a nucleic acid amplification method with the cartridge according to the present invention. The method includes (1) generating negative pressure in the reaction vessel by a suction from the exhaust port and thereby supplying the reaction solution containing nucleic acid to be amplified from the reaction solution supply port to the reaction solution supply space, and (2) subjecting the reaction solution that has spontaneously moved from the reaction solution supply space to the reaction solution storage space, to a nucleic acid amplification reaction.

In step (1), the reaction solution containing nucleic acid to be amplified is supplied from the reaction solution supply port to the reaction solution supply space. The supply of the reaction solution is performed by generating negative pressure in the reaction vessel by a suction from the exhaust port. The supply of the reaction solution to the reaction solution supply space is performed from the reaction solution supply port 32 positioned below the exhaust port 24 and above the protrusion 25, whereby the generation of bubble of the reaction solution can be suppressed, and the occurrence of the false positive can be further suppressed in the detection after the nucleic acid amplification. Furthermore, it is preferable that the amount of the reaction solution supplied to the reaction solution supply space is a volume corresponding to the volume of the reaction solution storage space. As a result, the reaction solution is easily stored in the reaction solution storage space in a single mass by the interaction of the protrusion, and the occurrence of the false positive in detection after nucleic acid amplification can be further suppressed.

In step (2), the reaction solution that has spontaneously moved from the reaction solution supply space to the reaction solution storage space is subjected to a nucleic acid amplification reaction. The reaction solution contains a nucleic acid to be amplified and additionally contains an enzyme for nucleic acid amplification and a primer or probe for nucleic acid amplification. The enzyme and primer or probe can be added to the reaction solution prior to its supply to the reaction vessel in advance or can be also added to it in the reaction vessel. The addition in the reaction vessel can be performed by placing a water-soluble bead containing the enzyme for nucleic acid amplification and a water-soluble bead containing the primer or probe for nucleic acid amplification between the reaction solution supply port and the protrusion in the reaction solution supply space.

The nucleic acid to be amplified in the reaction solution is not particularly limited and can be appropriately selected according to the purpose of use of the cartridge of the present invention. Furthermore, the enzyme for nucleic acid amplification and the primer and probe for nucleic acid amplification are not particularly limited, and an appropriate material according to the nucleic acid amplification method can be appropriately used.

The nucleic acid to be amplified is not particularly limited but may be, for example, RNA or DNA. The nucleic acid amplification reaction can be an isothermal amplification reaction or a thermocycle amplification reaction of nucleic acid. The isothermal amplification reaction is, for example, a LAMP method or a SmartAmp method. The thermocycle amplification reaction can be a PCR amplification reaction.

The enzyme for nucleic acid amplification is not particularly limited but may be, for example, an enzyme for the isothermal amplification reaction of nucleic acid or an enzyme for the thermocycle amplification reaction. The isothermal amplification reaction of nucleic acid can be performed by, for example, the LAMP method or SmartAmp method for nucleic acid and can be a nucleic acid amplification reaction with enzyme utilizing a strand displacement reaction.

A known polymerase, which is an enzyme for nucleic acid amplification reactions having a strand displacement activity, can be used as the enzyme. Examples of polymerase include that described in International Publication WO 2004/040019, but there is no intend to be limited thereto. The polymerase having the strand displacement activity can also be DNA polymerase (Aac), which is disclosed in International Publication WO 2009/054510 (JP patent 4450867).

Any polymerase that is used for the nucleic acid amplification reaction having the strand displacement activity can be used appropriately, whether it is normal temperature type, mesophilic, or thermostable. In addition, the polymerase can be either its natural form or a mutant form that is artificially mutated. Examples of such an polymerase include DNA polymerase. Examples of such DNA polymerase include mutants lacking 5'→3' exonuclease activity of DNA polymerases derived from thermophilic Bacillus bacteria such as Bacillus stearothermophilus (hereinafter referred to as "B. st") and Bacillus caldotenax (hereinafter referred to as "B. ca"), and the Klenow fragment of DNA polymerase I derived from escherichia coli (E. coli), and the like. Examples of DNA polymerase used in nucleic acid amplification reactions further include Vent DNA polymerase, Vent (Exo-) DNA polymerase, Deep Vent DNA polymerase, Deep Vent (Exo-) DNA polymerase, Φ29 phage DNA polymerase, MS-2 phage DNA polymerase, Z-Taq DNA polymerase, Pfu DNA polymerase, Pfu turbo DNA polymerase, KOD DNA polymerase, 9°Nm DNA polymerase, Therminator DNA polymerase, Taq DNA polymerase, and the like.

If the nucleic acid to be amplified is RNA, a reverse transcriptase can be used in addition to DNA polymerase, or a DNA polymerase having a reverse transcriptase activity can be used as DNA polymerase. The reverse transcriptase is not particularly limited as long as it has cDNA synthetic activity using RNA as a template, and examples thereof include reverse transcriptases of various origins, such as avian myeloblastosis virus-derived reverse transcriptase (AMVRTase), Rous-associated virus 2 reverse transcriptase (RAV-2RTase), and Moloney murine leukemia virus-derived reverse transcriptase (MMLV RTase). Examples of DNA polymerase having also the reverse transcriptase activity include BcaBEST DNA polymerase, Bca (exo-) DNA polymerase, Tth DNA polymerase, and the like.

The primer is appropriately selected according to the enzyme for nucleic acid amplification reaction. When the enzyme for nucleic acid amplification reaction is for a nucleic acid amplification reaction using the strand displacement reaction, examples of the primer include primers described in International Publication WO2004/040019, Japan Patent Publication JP2009-171935, Japan Patent Publication JP2011-50380 and the like.

The method of the present invention can further comprise a step of detecting the nucleic acid amplified in the reaction vessel after the nucleic acid amplification operation, by optically or electrically or by surface plasmon resonance. When a fluorogenic primer is used as the primer, the detection of the amplified nucleic acid can be performed by utilizing the label of the fluorogenic primer. When an exciton primer or an exciton probe is used in the amplification reaction, the detection of the amplified nucleic acid can be performed by utilizing the exciton effect. The method for optically detecting nucleic acid may be also a method utilizing an intercalating dye.

In the method of the present invention, it is preferable to perform the nucleic acid amplification reaction by the SmartAmp method or LAMP method and to detect the label with the exciton primer or exciton probe. Alternatively, it is preferable to perform the nucleic acid amplification reaction by the PCR method and to detect the label with the exciton primer or exciton probe.

Following the nucleic acid amplification reaction, a nucleic acid melting curve can be drawn, and the melting curve can be used to determine properties of the amplified product, such as whether it is a false positive or a true positive.

### INDUSTRIAL APPLICABILITY

The present invention is useful in fields related to the amplification and detection of nucleic acid.

### REFERENCE SIGNS LIST

10 Cartridge
20 Reaction vessel
21 Reaction solution storage space
22 Reaction solution supply space
23 Pillar
24 Exhaust port
25 Protrusion
26 Opening
30, 31 Flow channel
32 Reaction solution supply port

## Claims

1. A reaction vessel for nucleic acid amplification with a vertically long shape, including a reaction solution storage space, which is a lower portion thereof in a longitudinal direction, and a reaction solution supply space, which is an upper portion thereof, and comprising a reaction solution supply port and an exhaust port in the reaction solution supply space,
wherein the reaction solution supply port is positioned below the exhaust port in the longitudinal direction.

2. The reaction vessel according to claim 1, used for a method in which the reaction solution is supplied from the reaction solution supply port to the reaction solution supply space by a suction from the exhaust port to the outside.

3. A reaction vessel for nucleic acid amplification with a vertically long shape, including a reaction solution storage space, which is a lower portion thereof in a longitudinal direction, and a reaction solution supply space, which is an upper portion thereof, and comprising a protrusion toward the inside of the reaction vessel, in the vicinity of at least a part of the boundary between the reaction solution supply space and the reaction solution storage space.

4. The reaction vessel according to claim 1 or 2, wherein the reaction vessel comprises a protrusion toward the inside of the reaction vessel, in the vicinity of at least a part of the boundary between the reaction solution supply space and the reaction solution storage space.

5. The reaction vessel according to claim 3 or 4, wherein the protrusion is present along the entire boundary.

6. The reaction vessel according to any one of claims 3 to 5, wherein a ratio of a horizontal cross-sectional area at the position where the protrusion is provided is in the range of 30 to 95 when a horizontal cross-sectional area of the reaction vessel is set to 100.

7. The reaction vessel according to any one of claims 3 to 6, wherein an opening area in a horizontal cross-section at the position where the protrusion is provided is 1 to 10 mm².

8. The reaction vessel according to any one of claims 4 to 7, in the case that the reaction vessel has an exhaust port in the reaction solution supply space,
the reaction solution supply port is positioned below the exhaust port and above the protrusion.

9. The reaction vessel according to any one of claims 1 to 8, wherein the reaction solution storage space has a volume in the range of 10 to 500 µL.

10. The reaction vessel according to any one of claims 4 to 9, in the case that the reaction vessel has an exhaust port in the reaction solution supply space,
L1:L2:L3 ratio can be, for example, in the range of 1:0.1 to 1:0.1 to 1, wherein L1 is a distance between the exhaust port and the reaction solution supply port, L2 is a distance between the protrusion and the reaction solution supply port, and L3 is a distance between the protrusion and the bottom of the reaction solution storage space, and when L1+L2+L3 is set to 1, L2+L3 is, for example, in the range of 0.3 to 0.7.

11. The reaction vessel according to any one of claims 1 to 10, wherein at least a part of the side wall of the reaction solution storage space has light transparency.

12. The reaction vessel according to any one of claims 1 to 11, wherein the reaction solution storage space has a horizontal cross-sectional area smaller than a horizontal cross-sectional area of the reaction solution supply space.

13. The reaction vessel according to any one of claims 1 to 12, wherein at least a part or all of the inner surface of the reaction vessel has a surface roughness Ra of 25 nm or less.

14. The reaction vessel according to any one of claims 1 to 12, wherein at least a part or all of the inner surface of the reaction vessel is coated with wax.

15. A cartridge (10) for nucleic acid amplification comprising chambers in communication via a micro flow channel, and at least one reaction vessel (20) for nucleic acid amplification in communication with the chambers via a micro flow channel (30 and 31),
wherein the reaction vessel (20) is the reaction vessel according to any one of claims 1 to 14.

16. A nucleic acid amplification method with the cartridge according to claim 15, comprising:
generating negative pressure in the reaction vessel by a suction from the exhaust port and thereby supplying the reaction solution containing nucleic acid to be amplified from the reaction solution supply port to the reaction solution supply space,
subjecting the reaction solution that has spontaneously moved from the reaction solution supply space to the reaction solution storage space, to a nucleic acid amplification reaction.
